# FASCICULE DE BREVET EUROPEEN

(11) **EP 1 024 780 B1**
(45) Date de publication et mention de la délivrance du brevet: **07.12.2005**
(21) Numéro de dépôt: 98947611.4
(22) Date de dépôt: 06.10.1998
(51) Int. Cl.: A61K 7/09

(54) **AGENT REDUCTEUR A PLUSIEURS COMPOSANTS COMPRENANT AU MOINS UNE COMPOSITION SOUS FORME DE POUDRE CONTENANT UN REDUCTEUR DES LIAISONS DISULFURES DU CHEVEU ET PROCEDE DE DEFORMATION PERMANENTE DES MATIERES KERATINIQUES**
REDUZIERENDES MITTEL ENTHALTEND EINE PULVERFÖRMIGE, DIE DISULFID-BRÜCKEN DES HAARES SPALTENDE ZUSAMMENSETZUNG UND VERFAHREN ZUR PERMANENTEN VERFORMUNG KERATINISCHER SUBSTANZEN
REDUCING AGENT CONSISTING OF SEVERAL COMPONENTS COMPRISING AT LEAST ONE COMPOSITION IN FORM OF A POWDER COMPRISING A REDUCING AGENT FOR THE DISULFIDE BONDS OF HAIR AND PROCESS FOR THE PERMANENT DEFORMATION OF KERATINIC SUBSTANCES

(30) Priorité: 10.10.1997 FR 9712714
(43) Date de publication de la demande: 09.08.2000
(73) Titulaire: L'OREAL, 75008 Paris (FR)
(72) Inventeur: NGUYEN, Ly-Lan, F-94240 L'Hay-les-Roses (FR); SABBAGH, Anne, F-92500 Rueil-Malmaison (FR)
(74) Mandataire: Casalonga, Axel
(86) Numéro de dépôt international: PCT/FR1998/002132
(87) Numéro de publication internationale: WO 1999/018923

(56) Documents cités:
- EP-A- 0 638 550
- US-A- 4 898 726
- US-A- 4 992 267

## Description

La présente invention est relative à un agent réducteur à au moins deux composants comprenant une composition solide sous forme de poudre et une composition liquide destinés à être utilisés dans un procédé de traitement des matières kératiniques en particulier des cheveux, en vue d'obtenir une déformation permanente de ces dernières et aux procédés mettant en oeuvre cet agent.

L'une des techniques couramment utilisée dans le domaine cosmétique pour imprimer aux cheveux une forme durable consiste à procéder à la déformation des cheveux en mettant en oeuvre un agent de réduction puis un agent oxydant.

La technique la plus usuelle pour obtenir une déformation permanente des cheveux consiste dans un premier temps à réaliser l'ouverture des liaisons disulfure-S-S de la kératine (cystine) à l'aide d'une composition contenant un agent réducteur, puis après avoir de préférence rincé la chevelure ainsi traitée, à reconstituer dans un second temps lesdites liaisons disulfure en appliquant sur les cheveux préalablement mis sous tension par des bigoudis ou autres ou mis en forme ou lissés par d'autres moyens, une composition oxydante encore appelée "fixateur" de façon à donner, finalement, aux cheveux la forme recherchée.

Cette technique permet ainsi de réaliser, soit l'ondulation des cheveux, soit leur défrisage ou leur décrêpage ou encore leur lissage.

Les documents US 4 992 267 et US 4 898 726 décrivent notamment l'utilisation d'hydroxyde de sodium comme agent réducteur principal et d'un agent de défrisage auxiliaire, dans des conditions de pH compris entre 12 et 14, pour défriser les cheveux.

Cette nouvelle forme, imposée aux cheveux par un traitement chimique, est durable dans le temps pendant quelques semaines et résiste notamment à l'action des lavages à l'eau ou par des shampoings et ceci par opposition aux techniques mettant en oeuvre des produits de coiffage entraînant une déformation temporaire, telles que de mise en plis, cette déformation disparaissant cependant au coiffage ou aux shampoings.

Les compositions réductrices généralement utilisées pour la première étape d'une opération de permanente contiennent à titre d'agents réducteurs des sulfites, des bisulfites ou de préférence des thiols. Parmi ceux-ci, on peut citer plus particulièrement la cystéine et ses dérivés, la cystéamine et ses dérivés, l'acide thiolactique, l'acide thioglycolique ainsi que ces esters, notamment le thioglycolate de glycérol. L'acide thioglycolique est particulièrement efficace et constitue le produit le plus utilisé pour réduire les liaisons disulfures de la kératine.

On constate toutefois que les compositions contenant les réducteurs, du fait des propriétés réductrices de ces composants, présentent l'inconvénient d'avoir une mauvaise stabilité en solution. On constate pour de nombreux réducteurs notamment des dépôts blancs dans le fond des récipients contenant les compositions réductrices ainsi que le développement d'une odeur désagréable de sulfure d'hydrogène.

Ces évolutions s'accompagnent de la diminution en titre en réducteurs donc de leur efficacité.

Le parfumage des milieux réducteurs est particulièrement difficile en raison de la dégradation importante, lors du stockage, des parfums par ces agents réducteurs.

La présente invention permet de résoudre ces problèmes en mettant à disposition un agent réducteur à deux composants comprenant un premier composant constitué par une composition solide sous forme de poudre contenant un agent réducteur de la kératine et un second composant constitué par un liquide sans réducteur de la kératine. La préparation de la composition réductrice s'effectue de façon extemporanée par mélange des compositions tout juste avant l'application sur les fibres kératiniques.

Un objet de l'invention est donc constitué par un agent réducteur à aux moins deux composants comprenant une composition sous forme de poudre contenant un agent réducteur.

Un autre objet de l'invention est constitué par un procédé de déformation permanente des cheveux mettant en oeuvre un agent réducteur résultant de l'utilisation d'une composition sous forme de poudre mélangée à un liquide au moment de l'emploi.

D'autres objets de l'invention apparaîtront à la lecture de la description et des exemples qui suivent.

La composition solide sous forme de poudre de l'agent réducteur conforme à l'invention est essentiellement caractérisée par le fait qu'elle comprend au moins un agent réducteur de la kératine sous forme solide ou adsorbé sur un solide.

A titre d'exemples, les agents réducteurs solides utilisables selon l'invention sont les sulfites, la cystéine, la cystéamine ou l'un de leurs sels cosmétiquement acceptables tels que les chlorhydrates, bromhydrates, citrates, acétates, sulfates.

Parmi les réducteurs adsorbables sur des poudres, on peut citer à titre d'exemples l'acide thioglycolique, l'acide thiolactique, ou leurs sels, les esters d'acide thioglycolique tels que le thioglycolate de glycérol ou les esters d'acide thiolactique.

Les solides pouvant servir de support d'adsorption sont par exemple les silices, des argiles, des carbohydrates comme les amidons; des polymères organiques comme le nylon.

Le composé réducteur est utilisé dans des proportions telles que dans la composition réductrice au moment de l'emploi, il soit présent dans des quantités suffisantes pour réduire les liaisons -S-S- et de préférence comprises entre 1 et 25%, en particulier de 3 à 25% en poids.

La composition contient de préférence des agents épaississants sous forme de poudre et de préférence des agents épaississants dérivés de substances naturelles telles que plus particulièrement la gomme de guar, la gomme de tara, la farine d'epicéa ou des épaississants synthétiques tels que des polymères d'acide acrylique ou méthacrylique comme le copolymère d'acide méthacrylique/méthacrylate de méthyle comme celui commercialisé sous la dénomination Rohagit HSV (copolymère non réticulé acide méthacrylique/méthacrylate de méthyle (33/67).

La composition peut également contenir d'autres additifs habituellement utilisés dans les compositions réductrices et n'interférant pas avec les propriétés réductrices de la composition tels que des polymères sous forme de poudre, des agents alcalins ou acidifiants sous forme de poudre tels que plus particulièrement l'arginine ou l'acide citrique.

Dans le cadre du procédé conforme à l'invention, cette composition sous forme de poudre est utilisée conjointement avec une composition liquide sans réducteur de la kératine contenant dans un milieu aqueux constitué par de l'eau ou un mélange eau/solvant cosmétiquement acceptable, des adjuvants habituellement utilisés dans des compositions réductrices.

Le pH des compositions liquides est ajusté de façon à avoir un pH de la composition réductrice prête à l'emploi, généralement compris entre 6,5 et 11,5.

Les agents alcalins peuvent être choisis parmi la monoéthanolamine, la diéthanolamine, la triéthanolamine, l'isopropanolamine, le 2-méthyl 2-aminopropanol 1, la propanediamine-1,3, un carbonate de bicarbonate alcalin ou d'ammonium, l'ammoniaque, un carbonate organique tel que le carbonate de guanidine ou encore un hydroxyde alcalin, utilisés seul ou en mélange.

L'agent réducteur peut également contenir, soit dans la partie poudre, soit dans la partie liquide, soit dans le mélange poudre-liquide final, des agents tensio-actifs, des agents traitants de nature anionique, non-ionique, amphotère ou cationique.

Les agents tensio-actifs utilisés peuvent être de type non-ionique, anionique, cationique ou amphotère, couramment utilisés dans les compositions réductrices de permanentes. Parmi ceux-ci, on peut citer les alkylsulfates, les alkylbenzènesulfates, les alkyléthersulfates, les alkylsulfonates, les sels d'ammonium quaternaire, les alkylbétaïnes, les alkylphénols oxyéthylénés, les alcanolamides d'acides gras, les esters d'acides gras oxyéthylénés ainsi que des tensio-actifs non-ioniques de la famille des hydroxypropyléthers.

Ces agents tensio-actifs sont généralement utilisés dans des proportions telles que dans la composition résultant du mélange de la poudre et du liquide,leur proportion maximale soit de l'ordre de 30% en poids, et de préférence comprise entre 0,5 et 10% en poids par rapport au poids total de la composition.

On peut utiliser comme agents traitants des silicones volatiles ou non, linéaires ou cycliques ou leurs mélanges, les polydiméthylsiloxanes, les polyorganosiloxanes quaternisés, ceux décrits dans la demande de brevet français 2 535 730, les polyorganosiloxanes à groupement amino-alkyle modifiés par des groupements alcoxycarbonylalkyle tels que décrits dans le brevet US-A-4 749 732, les polyorganosiloxanes tels que les copolymères de polydiméthylsiloxane-polyoxyalkyle tel que le diméthicone copolyol, un polydiméthylsiloxane à groupements terminaux stéaroxy-(stéaroxydiméthicone), un copolymère polydiméthylsiloxane dialkylammonium acétate ou un copolymère polydiméthylsiloxane polyalkylbétaïne décrit dans GB-A-2 197 352, des polysiloxanes organomodifiés par des groupements mercapto ou mercapto-alkyle tels que décrits dans FR-B-1 530 369 et EP-A-0 295 780, ainsi que des silanes tels que le stéaroxy-triméthylsilane.

On peut également utiliser d'autres ingrédients traitants tels que des cires, des polymères choisis parmi les polymères cosmétiquement acceptables qui peuvent être des polymères cationiques, anioniques, non-ioniques ou amphotères, des agents de gonflement et de pénétration permettant de renforcer l'efficacité du réducteur tels que le diméthylisosorbitol, l'urée et ses dérivés, la pyrrolidone, les n-alkylpyrrolidone, la thiamorpholinone, les alkyléthers d'allcylèneglyeol ou de dialkylèneglycol tels que par exemple le monométhyléther de propylèneglycol, le monométhyléther de dipropylèneglycol, des alcanediols en C₃-C₆ tels que par exemple le propanediol-1,2, l'imidazolidinone-2, ainsi que d'autres composés tels que des alcools gras, des dérivés de lanoline, des céramides et notamment des céramides elles-mêmes , les glycocéramides, les pseudo-céramides décrits notamment dans FR-A-95/1399, et dans DOWNING Journal of Lipid Research, Vol. 35, p. 2060, 1994, ou dans FR-A-2 673 179, EP-A-0227994, WO-94/07844, WO-92/05764, dont le contenu est inclu par référence, des ingrédients actifs tels que l'acide pantoténique, des agents anti-chute, des agents anti-pelliculaires, des agents de mise en suspension, des agents séquestrants, des agents opacifiants, des colorants, des filtres solaires, ainsi que des parfums et des conservateurs.

Au moment de l'emploi, on mélange la poudre contenant l'agent réducteur de la kératine avec la composition liquide sans réducteur de la kératine. Le rapport du mélange est compris entre 0,01 et 4 en poids, c'est-à-dire que l'on peut utiliser de 1 à 80% en poids par rapport au poids total de la composition finale de la composition sous forme de poudre et de 99% à 20% de la composition sous forme liquide.

Le procédé conforme à l'invention est mis en oeuvre en mélangeant, comme indiqué ci-dessus, la composition sous forme de poudre contenant l'agent réducteur avec la composition liquide dans les proportions indiquées ci-desssus. On applique la composition résultante après mélange sur une chevelure, de préférence mouillée au préalable.

Cette application peut être réalisée avant, pendant ou après l'étape éventuelle de mise sous tension des cheveux sous une forme correspondant à la forme finale désirée pour ces derniers, telle que des boucles.

Lorsqu'on utilise des agents épaississants avec la poudre, on peut mettre éventuellement le procédé en oeuvre sans réaliser la mise sous tension des cheveux, mais en appliquant simplement la composition à l'aide des doigts ou du peigne, ce qui permet de sculpter la chevelure et maintenir les cheveux dans une position désirée telle que boucles, crans, épis.

Selon une étape facultative du procédé de l'invention, on peut, après application de la composition réductrice, soumettre la chevelure à un traitement thermique par chauffage à une température comprise entre 30 et 60°C. Ce chauffage permet éventuellement d'ajuster le degré final de frisure du cheveu.

Dans la pratique, cette opération peut être conduite au moyen d'un casque de coiffure, d'un sèche-cheveux, d'un dispensateur de rayons infrarouges et d'autres appareils chauffants classiques.

Il est également possible de travailler à température ambiante.

De façon générale, avant de procéder au rinçage ou à l'application de la composition oxydante, on laisse reposer pendant quelques minutes, généralement entre 2 et 30 minutes, et de préférence entre 5 et 20 minutes, la chevelure sur laquelle a été appliquée la composition réductrice, ceci de façon à bien laisser le temps au réducteur d'agir correctement sur les cheveux. Pendant cette phase d'attente, on peut utiliser dans ce but des bonnets.

Après le rinçage éventuel, on applique sur les cheveux une composition de fixation contenant un agent oxydant. Il s'agit de compositions habituellement utilisées dans les procédés de déformation permanente des cheveux. Des agents oxydants utilisables sont des peroxydes tels que l'eau oxygénée ou éventuellement les péroxydes d'urée, les bromates tels que les bromates alcalins, les persels ou un mélange de bromates alcalins et d'un persel.

La concentration en eau oxygénée peut varier de 1 à 10 volumes, mais de préférence est de l'ordre de 8 volumes.

La concentration en bromates alcalins est de 1 à 12% et celle en persels de 0,1 à 15% en poids par rapport au poids total de la composition oxydante.

Le pH de la composition oxydante peut varier entre 2 et 9, et de préférence est compris entre 3 et 8.

L'eau oxygénée peut être stabilisée par exemple par la phénacétine, l'acétaniline, les phosphates mono- et trisodiques ou par les sulfates d'hydroxy-8 chinoléïne.

Les compositions fixatrices ou oxydantes peuvent également contenir des agents alcanisants ou acidifiants ou des agents conservateurs, des agents séquestrants, des opacifiants, des agents traitants tels que définis ci-dessus pour la composition réductrice.

On peut retirer de la chevelure les moyens mécaniques qui maintenaient les cheveux sous tension avant ou après l'étape de fixation.

En tout état de cause, on procède après un temps de pose de 5 à 30 minutes, en particulier de 5 à 15 minutes, à un rinçage abondant à l'eau des cheveux ainsi traités par la composition fixatrice ou oxydante.

Les exemples suivants sont destinés à illustrer l'invention sans présenter un caractère limitatif.

### EXEMPLE 1

### Partie poudre A :

- Cystéine 5 g

### Partie liquide B :

- Monoéthanolamine 2,6 g
- Parfum 0,5 g
- Alcool oléique oxyéthyléné (20 moles d'oxyde d'éthylène) 1 g
- Cocoyl amidopropyl diméthyl hydroxypropyl 2 g sulfobétaïne en solution aqueuse à 50%
- Acide diéthylène triamine pentacétique, sel 0,2 g pentasodique en solution aqueuse à 40%
- Eau déminéralisée qs 95 g

On mélange au moment de l'emploi la poudre A et la lotion B. On obtient un liquide réducteur de pH 9,1, agréablement parfumé.

Une fois les constituants de cette composition mélangés, on applique cette composition sur des cheveux mouillés préalablement enroulés sur des bigoudis. Après avoir laissé agir la composition pendant environ 15 minutes, on rince abondamment à l'eau, puis on applique la composition oxydante suivante :
- Eau oxygénée qsp 8 volumes pH 3

On laisse agir la composition oxydante pendant environ 5 minutes, puis on rince abondamment la chevelure à l'eau et on enlève les rouleaux.

Après séchage, sous casque, les cheveux présentent de belles boucles.

### EXEMPLE 2

### Partie poudre A :

- Cystéine 3 g
- Farine de Tara 2 g
- Farine d'épicéa 5 g

### Partie liquide B :

- Monoéthanolamine 2,2 g
- Parfum 0,5 g
- Alcool oléique oxyéthyléné (20 moles d'oxyde d'éthylène) 1 g
- Mélange cocoyl amidopropyl bétaïne/monolaurate de glycérol en solution aqueuse à 30% 1,8 g
- Acide diéthylène triamine pentacétique, 0,2 g sel pentasodique en solution aqueuse à 40%
- Homopolymère chlorure de diméthyl diallyl 2,5 g ammonium en solution aqueuse à 40%
- Eau déminéralisée qs 90 g

Au moment de l'emploi, on mélange la poudre A et la lotion B. Le mélange est très facile à réaliser et on obtient très rapidement un réducteur épaissi permettant des applications sur cheveux déroulés avec d'excellentes qualités d'usage (application agréable, absence d'odeur désagréable, pas de coulures, rinçage facile, ...). Le pH du produit est 9,1.

On laisse poser ce réducteur épaissi 15 minutes. On rince. On fixe avec la composition oxydante décrite dans l'exemple 1 pendant 5 minutes. On rince abondamment et on sèche les cheveux.

## Revendications

1. Agent destiné à être utilisé comme réducteur dans un procédé de déformation de permanente des cheveux, **caractérisé par le fait qu'**il comprend au moins :
- un premier composant constitué par une composition sous forme de poudre, comprenant au moins un agent réducteur de la kératine solide ou adsorbé sur un solide, et
- un second composant constitué par une composition liquide aqueuse ne contenant pas de réducteur de la kératine,
les différents composants étant destinés à être mélangés l'un avec l'autre au moment de l'emploi en vue d'obtenir une composition réductrice présentant un pH compris entre 6,5 et 11,5 destinée à être appliquée sur des cheveux en vue de réduire les liaisons disulfures de ceux-ci.

2. Agent selon la revendication 1, **caractérisé par le fait que** le réducteur de la kératine solide est choisi parmi les sulfites, les thiols solides ou l'un de leurs sels cosmétiquement acceptables.

3. Agent selon la revendication 2, **caractérisé par le fait que** le thiol solide est choisi parmi la cystéine ou la cystéamine.

4. Agent selon la revendication 2 ou 3, **caractérisé par le fait que** les sels cosmétiquement acceptables sont choisis parmi les chlorhydrates, bromhydrates, citrates, acétates, sulfates.

5. Agent selon la revendication 1, **caractérisé par le fait que** l'agent réducteur de la kératine solide est choisi parmi des réducteurs de la kératine adsorbés sur des particules solides.

6. Agent selon la revendication 5, **caractérisé par le fait que** les réducteurs adsorbés sur les particules solides sont choisis parmi l'acide thioglycolique, l'acide thiolactique, leurs sels ou les esters d'acide thioglycolique ou les esters d'acide thiolactique.

7. Agent selon la revendication 5 ou 6, **caractérisé par le fait que** les particules solides sur lesquelles le réducteur de la kératine est adsorbé sont choisies parmi les silices, les argiles, les carbohydrates, les polymères organiques.

8. Agent selon l'une quelconque des revendications 1 à 7, **caractérisé par le fait que** la composition sous forme de poudre contient en plus au moins un agent épaississant choisi parmi la gomme de guar, la gomme de tara, la farine d'epicéa.

9. Agent selon l'une quelconque des revendications 1 à 8, **caractérisé par le fait que** la composition sous forme de poudre contient en plus des polymères en poudre, un ou plusieurs agents alcalins, un ou plusieurs agents acides.

10. Agent selon l'une quelconque des revendications 1 à 9, **caractérisé par le fait que** l'agent réducteur est présent dans des proportions telles que la concentration en agent réducteur dans la composition prête à l'emploi soit comprise entre 1 et 25% en poids par rapport au poids total de la composition.

11. Procédé de déformation de permanente des cheveux, **caractérisé par le fait**
- **qu'**on procède au préalable à un mélange d'une composition sous forme de poudre contenant au moins un agent réducteur solide ou adsorbé sur un solide avec une composition liquide sans réducteur de la kératine;
- **qu'**on applique cette composition présentant un pH compris entre 6,5 et 11,5 sur la chevelure avant, pendant ou après l'étape éventuelle de mise sous tension des cheveux;
- **qu'**on applique après un temps de pose suffisant pour permettre la réduction des liaisons disulfures du cheveu, une composition de fixation contenant au moins un agent oxydant;
- **qu'**on procède au rinçage après un temps de pose suffisant pour permettre la déformation permanente.

12. Procédé selon la revendication 11, **caractérisé par le fait que** la composition sous forme de poudre contient un agent réducteur de la kératine tel que défini dans l'une quelconque des revendications 2 à 6.

13. Procédé selon la revendication 11 ou 12, **caractérisé par le fait que** le rapport du mélange entre la composition sous forme de poudre et la composition liquide, est compris entre 0,01 et 4 exprimé en poids.

14. Procédé selon l'une quelconque des revendications 11 à 13, **caractérisé par le fait que** l'on mélange 1 à 80% en poids de composition sous forme de poudre avec 99 à 20% de composition liquide.

15. Procédé selon l'une quelconque des revendications 11 à 14, **caractérisé par le fait que** la composition réductrice contient des agents tensio-actifs anioniques, non-ioniques, cationiques ou amphotères ajoutés, soit dans la composition liquide ou directement dans la composition prête à l'emploi.

16. Procédé selon la revendication 15, **caractérisé par le fait que** les agents tensio-actifs sont présents dans des proportions telles que la composition prête à l'emploi en contienne au maximum 30% en poids.

17. Procédé selon l'une quelconque des revendications 11 à 16, **caractérisé par le fait que** la composition prête à l'emploi contient des agents traitants choisis parmi les silicones, des cires, des polymères, des agents de gonflement et de pénétration, des alcools gras, des dérivés de lanoline, des céramides, des ingrédients actifs, des agents anti-chute, anti-pelliculaires, des agents de mise en suspension, des agents séquestrants, des agents opacifiants, des colorants, des filtres solaires,des conservateurs.

18. Procédé selon l'une quelconque des revendications 11 à 17, **caractérisé par le fait que** la composition réductrice est maintenue au contact des cheveux pendant 2 à 30 minutes.

19. Procédé selon la revendication 11, **caractérisé par le fait que** les cheveux sont rincés avant l'application de la composition oxydante.

20. Procédé selon l'une quelconque des revendications 11 à 19, **caractérisé par le fait que** la composition oxydante contient un agent oxydant choisi parmi le peroxyde d'hydrogène, le peroxyde d'urée, les bromates, les persels ou leurs mélanges.

21. Procédé selon l'une quelconque des revendications 11 à 20, **caractérisé par le fait que** l'on maintient la composition oxydante au contact des cheveux et que l'on rince ensuite à l'eau.

## Patentansprüche

1. Mittel, das in einem Verfahren zur dauerhaften Verformung der Haare als Reduktionsmittel verwendet werden soll, **dadurch gekennzeichnet, dass** es zumindest umfasst:
- eine erste Komponente, die aus einer Zusammensetzung in Pulverform besteht, die mindestens ein festes oder an einem Feststoff adsorbiertes Reduktionsmittel für das Keratin enthält, und
- eine zweite Komponente, die aus einer flüssigen wässrigen Zusammensetzung besteht, die kein Reduktionsmittel für das Keratin enthält,
wobei die verschiedenen Komponenten bei der Anwendung miteinander vermischt werden sollen, um eine reduzierende Zusammensetzung mit einem pH-Wert von 6,5 bis 11,5 herzustellen, die auf die Haare aufgetragen werden soll, um die Disulfidbindungen der Haare zu reduzieren.

2. Mittel nach Anspruch 1, **dadurch gekennzeichnet, dass** das feste Reduktionsmittel für das Keratin unter den Sulfiten, festen Thiolen oder deren kosmetisch akzeptablen Salzen ausgewählt ist.

3. Mittel nach Anspruch 2, **dadurch gekennzeichnet, dass** das feste Thiol unter Cystein oder Cysteamin ausgewählt ist.

4. Mittel nach Anspruch 2 oder 3, **dadurch gekennzeichnet, dass** die kosmetisch akzeptablen Salze unter den Hydrochloriden, Hydrobromiden, Citraten, Acetaten und Sulfaten ausgewählt sind.

5. Mittel nach Anspruch 1, **dadurch gekennzeichnet, dass** das feste Reduktionsmittel für das Keratin unter den Reduktionsmitteln für das Keratin ausgewählt ist, die an festen Partikeln adsorbiert sind.

6. Mittel nach Anspruch 5, **dadurch gekennzeichnet, dass** die an festen Partikeln adsorbierten Reduktionsmittel unter Thioglykolsäure, Thiomilchsäure, deren Salzen oder den Estern von Thioglykolsäure oder den Diestern von Thiomilchsäure ausgewählt sind.

7. Mittel nach Anspruch 5 oder 6, **dadurch gekennzeichnet, dass** die festen Partikel, an denen das Reduktionsmittel für das Keratin adsorbiert ist, unter den Kieselsäuren, Tonen, Kohlenhydraten und organischen Polymeren ausgewählt ist.

8. Mittel nach einem der Ansprüche 1 bis 7, **dadurch gekennzeichnet, dass** die pulverförmige Zusammensetzung ferner mindestens ein Verdickungsmittel enthält, das unter Guargummi, Taragummi und Fichtenmehl ausgewählt ist.

9. Mittel nach einem der Ansprüche 1 bis 8, **dadurch gekennzeichnet, dass** die pulverförmige Zusammensetzung ferner pulverförmige Polymere, ein oder mehrere Alkalisierungsmittel oder ein oder mehrere Ansäuerungsmittel enthält.

10. Mittel nach einem der Ansprüche 1 bis 9, **dadurch gekennzeichnet, dass** das Reduktionsmittel in solchen Mengenanteilen enthalten ist, dass die Konzentration des Reduktionsmittels in der gebrauchsfertigen Zusammensetzung im Bereich von 1 bis 25 Gew.-%, bezogen auf das Gesamtgewicht der Zusammensetzung, liegt.

11. Verfahren zur dauerhaften Verformung der Haare, **dadurch gekennzeichnet, dass**
- vorab eine pulverförmige Zusammensetzung, die mindestens ein festes oder an einem Feststoff adsorbiertes Reduktionsmittel enthält, mit einer flüssigen Zusammensetzung vermischt wird, die kein Reduktionsmittel für das Keratin enthält;
- diese Zusammensetzung, die einen pH-Wert im Bereich von 6,5 bis 11 aufweist, vor, während oder nach dem gegebenenfalls durchgeführten Schritt, bei dem die Haare unter Spannung gesetzt werden, aufgetragen wird;
- nach einer Einwirkzeit, die ausreichend ist, damit die Disulfidbindungen der Haare reduziert werden, eine fixierende Zusammensetzung aufgetragen wird, die mindestens ein Oxidationsmittel enthält; und
- nach einer für die permanente Verformung ausreichenden Einwirkzeit gespült wird.

12. Verfahren nach Anspruch 11, **dadurch gekennzeichnet, dass** die pulverförmige Zusammensetzung ein Reduktionsmittel für das Keratin nach einem der Ansprüche 2 bis 6 enthält.

13. Verfahren nach Anspruch 11 oder 12, **dadurch gekennzeichnet, dass** das Verhältnis der pulverförmigen Zusammensetzung und der flüssigen Zusammensetzung in dem Gemisch, auf das Gewicht bezogen ausgedrückt, im Bereich von 0,01 bis 4 liegt.

14. Verfahren nach einem der Ansprüche 11 bis 13, **dadurch gekennzeichnet, dass** 1 bis 80 Gew.-% der pulverförmigen Zusammensetzung mit 99 bis 20 Gew.-% der flüssigen Zusammensetzung vermischt werden.

15. Verfahren nach einem der Ansprüche 11 bis 14, **dadurch gekennzeichnet, dass** die reduzierende Zusammensetzung anionische, nichtionische, kationische oder amphotere grenzflächenaktive Stoffe enthält, die entweder in die flüssige Zusammensetzung oder direkt in die gebrauchsfertige Zusammensetzung gegeben werden.

16. Verfahren nach Anspruch 15, **dadurch gekennzeichnet, dass** die grenzflächenaktiven Stoffe in solchen Mengenanteilen enthalten sind, dass die gebrauchsfertige Zusammensetzung maximal 30 Gew.-% grenzflächenaktive Stoffe enthält.

17. Verfahren nach einem der Ansprüche 11 bis 16, **dadurch gekennzeichnet, dass** die gebrauchsfertige Zusammensetzung Behandlungsmittel enthält, die unter den Siliconen, Wachsen, Polymeren, Quellmitteln, Penetrationsmitteln, Fettalkoholen, Lanolinderivaten, Ceramiden, Wirkstoffen, Wirkstoffen gegen Haarausfall, Wirkstoffen gegen Schuppen, Suspendiermitteln, Maskierungsmitteln, Trübungsmitteln, Farbmitteln, Sonnenschutzfiltem und Konservierungsmitteln ausgewählt sind.

18. Verfahren nach einem der Ansprüche 11 bis 17, **dadurch gekennzeichnet, dass** die reduzierende Zusammensetzung 2 bis 30 Minuten mit dem Haar in Kontakt belassen wird.

19. Verfahren nach Anspruch 11, **dadurch gekennzeichnet, dass** die Haare vor dem Auftragen der oxidierenden Zusammensetzung gespült werden.

20. Verfahren nach einem der Ansprüche 11 bis 19, **dadurch gekennzeichnet, dass** die oxidierende Zusammensetzung ein Oxidationsmittel enthält, das unter Wasserstoffperoxid, Harnstoffperoxid, Bromaten, Salzen von Persäuren oder deren Gemischen ausgewählt ist.

21. Verfahren nach einem der Ansprüche 11 bis 20, **dadurch gekennzeichnet, dass** die oxidierende Zusammensetzung mit den Haaren in Kontakt belassen und anschließend mit Wasser gespült wird.

## Claims

1. Agent intended to be used as a reducing agent in a process for permanently reshaping the hair, **characterized in that** it comprises at least:
- a first component consisting of a composition in powder form, comprising at least one keratin-reducing agent which is solid or adsorbed onto a solid, and
- a second component consisting of an aqueous liquid composition containing no keratin-reducing agent,
the various components being intended to be mixed with each other at the time of use in order to obtain a reducing composition having a pH of between 6.5 and 11.5 and intended to be applied to the hair in order to reduce the disulphide linkages of the hair.

2. Agent according to Claim 1, **characterized in that** the solid keratin-reducing agent is chosen from sulphites and solid thiols or one of their cosmetically acceptable salts.

3. Agent according to Claim 2, **characterized in that** the solid thiol is chosen from cysteine and cysteamine.

4. Agent according to Claim 2 or 3, **characterized in that** the cosmetically acceptable salts are chosen from the hydrochlorides, hydrobromides, citrates, acetates and sulphates.

5. Agent according to Claim 1, **characterized in that** the solid keratin-reducing agent is chosen from keratin-reducing agents adsorbed onto solid particles.

6. Agent according to Claim 5, **characterized in that** the reducing agents adsorbed onto solid particles are chosen from thioglycolic acid, thiolactic acid, their salts, or thioglycolic acid esters or thiolactic acid esters.

7. Agent according to Claim 5 or 6, **characterized in that** the solid particles onto which the keratin-reducing agent is adsorbed are chosen from silicas, clays, carbohydrates and organic polymers.

8. Agent according to any one of Claims 1 to 7, **characterized in that** the composition in powder form also contains at least one thickener chosen from guar gum, tara gum and spruce meal.

9. Agent according to any one of Claims 1 to 8, **characterized in that** the composition in powder form also contains powdered polymers, one or more alkaline agents or one or more acidic agents.

10. Agent according to any one of Claims 1 to 9, **characterized in that** the reducing agent is present in proportions such that the concentration of reducing agent in the ready-to-use composition is between 1 and 25% by weight relative to the total weight of the composition.

11. Process for permanently reshaping the hair, **characterized in that**
- a composition in powder form containing at least one reducing agent which is solid or adsorbed onto a solid is mixed beforehand with a liquid composition containing no keratin-reducing agent;
- this composition having a pH of between 6.5 and 11.5 is applied to the hair before, during or after the optional step of placing the hair under tension;
- after an exposure time which is sufficient to allow reduction of the disulphide linkages of the hair, a fixing composition containing at least one oxidizing agent is applied;
- after an exposure time which is sufficient to allow the permanent reshaping, the hair is rinsed.

12. Process according to Claim 11, **characterized in that** the composition in powder form contains a keratin-reducing agent as defined in any one of Claims 2 to 6.

13. Process according to Claim 11 or 12, **characterized in that** the ratio of the mixture between the composition in powder form and the liquid composition is between 0.01 and 4, expressed by weight.

14. Process according to any one of Claims 11 to 13, **characterized in that** 1 to 80% by weight of composition in powder form is mixed with 99 to 20% of liquid composition.

15. Process according to any one of Claims 11 to 14, **characterized in that** the reducing composition contains anionic, nonionic, cationic or amphoteric surfactants added either to the liquid composition or directly into the ready-to-use composition.

16. Process according to Claim 15, **characterized in that** the surfactants are present in proportions such that the ready-to-use composition contains not more than 30% by weight of them.

17. Process according to any one of Claims 11 to 16, **characterized in that** the ready-to-use composition contains treating agents chosen from silicones, waxes, polymers, swelling agents, penetrating agents, fatty alcohols, lanolin derivatives, ceramides, active ingredients, agents for preventing hair loss, antidandruff agents, suspending agents, sequestering agents, opacitiers, colorants, sunscreens and preserving agents.

18. Process according to any one of Claims 11 to 17, **characterized in that** the reducing composition is kept in contact with the hair for 2 to 30 minutes.

19. Process according to Claim 11, **characterized in that** the hair is rinsed before applying the oxidizing composition.

20. Process according to any one of Claims 11 to 19, **characterized in that** the oxidizing composition contains an oxidizing agent chosen from hydrogen peroxide, urea peroxide, bromates and persalts, or mixtures thereof.

21. Process according to any one of Claims 11 to 20, **characterized in that** the oxidizing composition is kept in contact with the hair, and rinsing is then carried out with water.
